# FASCICULE DE BREVET EUROPEEN

(11) **EP 0 789 009 B1**
(45) Date de publication et mention de la délivrance du brevet: **06.09.2000**
(21) Numéro de dépôt: 97400257.8
(22) Date de dépôt: 05.02.1997
(51) Int. Cl.: C07C 6/12

(54) **Procédé de dismutation et/ou de transalkylation d'hydrocarbures alkylaromatiques en présence de deux catalyseurs zéolithiques**
Verfahren zur Dismutation und/oder Transalkylierung von alkylaromatischen Kohlenwasserstoffen in Gegenwart von zwei zeolitischen Katalysatoren
Process for dismutation and/or transalkylation of alkylaromatic hydrocarbons in presence of two zeolitic catalysts

(30) Priorité: 09.02.1996 FR 9601608
(43) Date de publication de la demande: 13.08.1997
(73) Titulaire: INSTITUT FRANCAIS DU PETROLE, 92502 Rueil-Malmaison (FR)
(72) Inventeur: Benazzi, Eric, 78360 Montesson (FR); Alario, Fabio, 92200 Neuilly sur Seine (FR)

(56) Documents cités:
- DE-A- 2 558 035
- FR-A- 2 291 957
- FR-A- 2 303 782
- US-A- 3 506 731
- US-A- 5 210 356

## Description

L'invention concerne la dismutation d'hydrocarbures alkylaromatiques, et de préférence la dismutation du toluène pour produire du benzène et des xylènes, et/ou la transalkylation d'hydrocarbures alkylaromatiques, et de préférence la transalkylation du toluène et de triméthylbenzènes pour produire des xylènes, dans une zone réactionnelle comportant au moins deux lits catalytiques comportant chacun un catalyseur différent, l'un des catalyseurs contenant au moins une zéolithe de structure mordénite, au moins en partie sous forme acide, et l'autre catalyseur contenant au moins une zéolithe de structure mazzite, au moins en partie sous forme acide, chaque catalyseur contenant au moins une matrice et éventuellement au moins un élément choisi dans l'ensemble formé par les groupes lB et VIII de la classification périodique des éléments.

De nombreux catalyseurs de dismutation et de transalkylation à base de mordénite ont déjà été décrits dans l'art antérieur. Il en est ainsi dans le brevet US-A-3.506.731 où une mordénite sous forme hydrogène est utilisée ainsi que dans la demande de brevet français FR-A-2.367.533. Il en est encore ainsi dans le brevet US-A-3.281.483 qui fait mention de mordénites échangées essentiellement avec des ions argent ou nickel, ou bien dans le brevet US-A-3.780.121 qui fait état d'une mordénite échangée avec des métaux du groupe lB de la classification périodique des éléments et qui est caractérisée par un rapport atomique Si/AI compris entre 6 et 40, ou bien encore dans le brevet US-A-3.629.351 qui concerne également une mordénite contenant des ions des métaux du groupe lB, VA, VIA, IIA et VIII de la classification périodique des éléments.

Plus récemment le brevet US-A-5.210.356 revendique l'utilisation d'un catalyseur de dismutation du toluène comportant une zéolithe oméga modifiée par désalumination et chargée en nickel.

D'autre part, le brevet US-A-5.371.311 nous enseigne qu'un catalyseur comportant une zéolithe oméga, synthétisée en utilisant des cations alcalins et un agent organique comme structurant organique, puis modifiée par calcination sous air, échanges ioniques, calcination en présence de vapeur d'eau et finalement par un traitement par une solution aqueuse d'ions ammonium à bas pH, conduit à des propriétés physico-chimiques supérieures et à des performances catalytiques améliorées dans les réactions de conversion des hydrocarbures et en particulier de dismutation et/ou de transalkylation des alkylaromatiques.

De façon surprenante, l'association dans une zone réactionnelle d'au moins deux lits catalytiques, comportant chacun un catalyseur différent, l'un des catalyseurs contenant au moins une zéolithe de structure mordénite, au moins en partie sous forme acide, et l'autre catalyseur contenant au moins une zéolithe de structure mazzite, au moins en partie sous forme acide, chaque catalyseur contenant au moins une matrice et éventuellement au moins un élément choisi dans l'ensemble formé par les groupes lB et VIII de la classification périodique des éléments, conduit à des performances catalytiques améliorées dans les réactions de dismutation d'hydrocarbures alkylaromatiques tel que le toluène, et/ou de transalkylation d'hydrocarbures alkylaromatiques tels que le toluène et les triméthylbenzènes, par rapport aux catalyseurs de l'art antérieur.

L'invention concerne donc un procédé de dismutation et/ou de transalkylation d'hydrocarbures dans une zone réactionnelle comportant au moins deux lits catalytiques comportant chacun un catalyseur différent, l'un des catalyseurs contenant au moins une zéolithe de structure mordénite, de préférence une mordénite, au moins en partie, de préférence pratiquement totalement, sous forme acide, et l'autre catalyseur contenant au moins une zéolithe de structure mazzite, de préférence une zéolithe oméga, au moins en partie, de préférence pratiquement totalement, sous forme acide, chaque catalyseur contenant au moins une matrice et éventuellement au moins un élément choisi dans l'ensemble formé par les groupes lB et VIII de la classification périodique des éléments.

Les conditions opératoires sont généralement les suivantes : une température comprise entre 250 et 600°C, de préférence entre 330 et 500°C ; une pression comprise entre 10 et 60 bar, de préférence entre 20 et 45 bar ; une vitesse spatiale d'alimentation, exprimée en kilogramme de charge introduite par kilogramme de catalyseur et par heure, comprise entre 0,1 et 10 et de préférence entre 0,5 et 4 ; un rapport molaire hydrogène sur hydrocarbures compris entre 2 et 20 et de préférence entre 3 et 12.

L'invention concerne donc un procédé de dismutation et/ou de transalkylation d'hydrocarbures dans une zone réactionnelle comportant au moins deux lits catalytiques comportant chacun un catalyseur différent, l'un des catalyseurs comportant généralement entre 5 et 95%, de préférence entre 15 et 90% et de façon encore plus préférée entre 25 et 85% d'une zéolithe de structure mordénite et éventuellement entre 0,01 et 10%, de préférence entre 0,05 et 7%, et de façon encore plus préférée entre 0,10 et 5%, d'au moins un élément choisi dans l'ensemble formé par les groupes lB et VIII de la classification périodique des éléments, le complément à 100 % poids consistant généralement en la part de matrice dans ledit catalyseur, et l'autre catalyseur comportant généralement entre 5 et 95%, de préférence entre 15 et 90% et de façon encore plus préférée entre 25 et 85% d'une zéolithe de structure mazzite et éventuellement entre 0,01 et 10%, de préférence entre 0,05 et 7%, et de façon encore plus préférée entre 0,10 et 5%, d'au moins un élément choisi dans l'ensemble formé par les groupes lB et VIII de la classification périodique des éléments, le complément à 100 % poids consistant généralement en la part de matrice dans ledit catalyseur.

Pour chacun des catalyseurs, la matrice est choisie généralement parmi les éléments du groupe formé par les argiles (par exemple parmi les argiles naturelles telles que le kaolin ou la bentonite), la magnésie, les alumines, les silices, l'oxyde de titane, l'oxyde de bore, la zircone, les phosphates d'aluminium, les phosphates de titane, les phosphates de zirconium et les silice-alumines, de préférence parmi les éléments du groupe formé par les alumines et les argiles.

La zéolithe de type structural mazzite est généralement choisie dans le groupe formé par la zéolithe oméga, la mazzite, la zéolithe LZ-202, la zéolithe mazzite gallosilicate ou la zéolithe ZSM-4, de préférence la zéolithe oméga, dont le diamètre des pores principaux est d'environ 7,4 Å et qui posséde un réseau microporeux monodimensionnel ("Atlas of Zeolites Structure Types", W.M. Meier et D.H. Olson, 3ème Edition, 1992).

La zéolithe de type structural mazzite est au moins en partie, de préférence pratiquement totalement, sous forme acide, c'est-à-dire sous forme hydrogène (H⁺).

La zéolithe de type structural mazzite a généralement une teneur en sodium inférieure à 0,6 %, de préférence inférieure à 0,1% poids (par rapport à ladite zéolithe), comprend du silicium et au moins un élément T choisi dans le groupe formé par le gallium et l'aluminium, de préférence l'aluminium, et a un rapport molaire Si/T est compris entre 5 et 100 et de préférence entre 7 et 80.

La zéolithe de type structural mordénite est généralement choisie dans le groupe formé par la mordénite et la zéolithe LZ-211, de préférence la mordénite, dont le diamètre des pores principaux est d'environ 7 x 6,5 Å et qui posséde un réseau microporeux monodimensionnel ("Atlas of Zeolites Structure Types", W.M. Meier et D.H. Olson, 3ème Edition, 1992).

La zéolithe de type structural mordénite est au moins en partie, de préférence pratiquement totalement, sous forme acide, c'est-à-dire sous forme hydrogène (H⁺).

La zéolithe de type structural mordénite a généralement une teneur en sodium inférieure à 0,2 %, de préférence inférieure à 0,1 % poids (par rapport à ladite zéolithe), comprend du silicium et de l'aluminium et a un rapport molaire Si/AI qui est compris entre 5 et 100 et de préférence entre 7 et 80.

La zéolithe de type structural mordénite est soit à petits pores, soit à larges pores, et elle est aussi bien synthétisée en milieu hydroxyde que fluorure.

La zéolithe de type structural mordénite à petits pores possède une teneur pondérale en sodium par rapport au poids de mordénite sèche généralement comprise entre 4 et 6,5%, un rapport atomique Si/AI global généralement compris entre 4 et 7, un volume de maille élémentaire généralement compris entre 2,76 et 2,80 nm³ (avec 1 nm=10⁻⁹m) et n'adsorbe habituellement que des molécules de diamètre cinétique inférieur à environ 4,4x10⁻¹⁰m.

La zéolithe de type structural mordénite à larges pores, synthétisée en milieu OH⁻ ou bien en milieu F⁻, par exemple selon le brevet EP-A-0.427.579, se distingue de celle du type à petits pores par le fait qu'elle peut adsorber des molécules de diamètre cinétique supérieur à environ 6,6x10⁻¹⁰m, donc en particulier des molécules de benzène, que son rapport atomique Si/AI global est généralement compris entre 4,5 et 20.

Il peut être nécessaire d'amener le rapport Si/Al global de la zéolithe de type structural mordénite à des valeurs supérieures à celles obtenues à la synthèse et mentionnées ci-avant. Afin d'obtenir des zéolithes de type structural mordénite désaluminées dans une large gamme de rapports Si/AI, toute méthode connue de l'homme du métier peut être employée comme l'attaque acide directe ou bien au moins un cycle de désalumination comprenant au moins une calcination, en présence ou non de vapeur d'eau, de la forme NH₄⁺ de la zéolithe, suivie d'au moins une attaque acide. Dans le cas spécifique de la zéolithe de type structural mordénite à petits pores, il faut s'assurer que les traitements retenus ont bien conduit à une ouverture des canaux.

Pour la préparation de la zéolithe de type structural mordénite, la mise sous forme ammonium (NH₄+) s'effectue généralement soit sur une zéolithe de type structural mordénite, brute de synthèse, forme sodique, sur laquelle plusieurs échanges ioniques par des solutions concentrées de nitrate d'ammonium (10N) permettent d'obtenir une teneur en pondérale en sodium, par rapport au poids de zéolithe de type structural mordénite sèche, généralement inférieure à 2000 ppm, de préférence à 1000 ppm, et de manière encore plus préférée à 500 ppm, soit sur une zéolithe de type structural mordénite désaluminée, pour laquelle plusieurs échanges successifs au nitrate d'ammonium permettent d'obtenir la forme NH₄+ de ladite zéolithe.

Après ce traitement, la zéolithe de type structural mordénite modifiée subit généralement un traitement thermique destiné à décomposer au moins partiellement, de préférence pratiquement totalement, les cations ammonium présents au sein du réseau et à obtenir ainsi au moins partiellement, de préférence pratiquement totalement, la forme acide (H-M) de la zéolithe de type structural mordénite.

La zéolithe de type structural mordénite peut avoir subi des modifications post-synthèse comme par exemple une désalumination par au moins un traitement par au moins une solution comprenant au moins un sel de fluorosilicate tel que l'hexafluorosilicate d'ammonium, comme cela est décrit dans la demande de brevet européen EP-A-0.573.347 ou dans le brevet US-A-4.503.023, ou à l'aide d'au moins un composé des éléments choisis parmi les éléments des groupes IIA, llB ou IVA de la classification périodique des éléments, comme cela est décrit dans la demande de brevet européen EP-A-0.569.268 ou dans le brevet US-A-5.391.528.

La zéolithe de type structural mordénite, qu'elle soit sous forme sodium, ammonium ou hydrogène, peut être éventuellement soumise au dépôt d'au moins un élément choisi dans l'ensemble formé par les groupes lB et VIII de la classification périodique des éléments, de préférence choisi dans le groupe formé par l'argent, le nickel et le platine, et de façon encore plus préférée le nickel, et peut être mise en forme par toute technique connue de l'homme du métier.

La zéolithe de type structural mazzite est fabriquée par toute méthode connue de l'homme du métier, à partir de zéolithe de type structural mazzite brute de synthèse dont le rapport Si/T compris entre 3 et 5.

Il est généralement nécessaire d'amener le rapport Si/T de la zéolithe de type structural mazzite à une valeur supérieure à celle de la zéolithe de type structural mazzite brute de synthèse mentionnée ci-avant. Afin d'obtenir des zéolithes de type structural mazzite dans une large gamme de rapport Si/T, toute méthode connue de l'homme de l'art peut être utilisée, en particulier une désalumination selon le mode opératoire décrit dans le brevet US-A-4.780.436 dans le cas préféré où T est l'aluminium, c'est-à-dire que l'on procède à une étape de calcination sous flux d'air sec, qui a pour but d'éliminer le structurant organique occlus dans la microporosité de la zéolithe, à au moins un échange ionique par au moins une solution de NH₄NO₃, de manière à éliminer pratiquement tout cation alcalin, en particulier le sodium, présent en position cationique dans la zéolithe, puis à au moins un cycle de désalumination de la charpente, comportant au moins une calcination en présence de vapeur d'eau, à une température généralement comprise entre 550 et 850 °C, suivie d'au moins une attaque acide.

Dans le cas préféré où T est l'aluminium, la désalumination de la charpente, comportant au moins une étape de calcination sous vapeur d'eau et au moins une étape d'attaque en milieu acide de la zéolithe de type structural mazzite, peut être répété autant de fois qu'il est nécessaire pour obtenir la zéolithe de type structural mazzite désaluminée possédant les caractéristiques désirées. De même, suite au traitement de calcination sous vapeur d'eau plusieurs attaques acides successives, avec des solutions en acide de concentration différentes, peuvent être opérées.

La préparation de chacun des catalyseurs peut être effectuée selon toute méthode connue de l'homme du métier. Elle est décite ci-après en une description qui peut s'appliquer à chacun des catalyseurs.

En général, la préparation de chacun des catalyseurs est obtenue par mélange de la matrice et de la zéolithe puis par mise en forme. L'élément éventuel de l'ensemble formé par les groupe lB et VIII de la classification périodique des éléments peut être intoduit soit avant la mise en forme, ou bien lors du mélange, ou bien sur la zéolithe elle même avant de la mélanger, soit, de préférence, après la mise en forme. La mise en forme est généralement suivie d'une calcination, généralement à une température comprise entre 250 et 600 °C. L'élément éventuel de l'ensemble formé par les groupe lB et VIII de la classification périodique des éléments peut être intoduit après ladite calcination. Dans tous les cas, ledit élément est généralement déposé au choix soit, de préférence, pratiquement totalement sur la zéolithe, soit pratiquement totalement sur la matrice, soit en partie sur la zéolithe et en partie sur la matrice, ce choix s'effectuant, de la manière qui est connue de l'homme du métier, par les paramètres utilisés lors dudit dépôt, comme par exemple la nature du précurseur choisi pour effectuer ledit dépôt.

L'élément des groupes lB ou VIII, de préférence choisi dans le groupe formé par Ag, Ni et Pt, et de façon encore plus préférée Ni, peut également éventuellement être déposé sur le mélange zéolithe-matrice préalablement mis en forme par tout procédé connu de l'homme de l'art. Un tel dépôt est généralement effectué par la technique d'imprégnation à sec, d'échange(s) ionique(s) ou de coprécipitation. Dans le cas de l'échange ionique à partir de précurseurs à base d'argent, de nickel ou de platine, on utilise habituellement des sels d'argent tels que les chlorures ou les nitrates, un complexe tétramine du platine, ou des sels de nickel tels que les chlorures, les nitrates, les acétates ou les formiates. Cette technique d'échange cationique peut également être utilisée pour déposer directement le métal sur la poudre de zéolithe, avant son mélange éventuel avec une matrice.

Dans le cas où le catalyseur contient plusieurs métaux, ces derniers peuvent être introduits soit tous de la même façon soit par des techniques différentes, avant ou après mise en forme et dans n'importe quel ordre. Dans le cas où la technique utilisée est celle de l'échange ionique, plusieurs échanges successifs peuvent être nécessaires pour introduire les quantités requises de métaux.

Par exemple, une des méthodes préférées de préparation du catalyseur selon l'invention consiste à malaxer la zéolithe dans un gel humide de matrice (obtenu généralement par mélange d'au moins un acide et d'une poudre de matrice), par exemple d'alumine, pendant une durée nécéssaire pour l'obtention d'une bonne homogénéité de la pâte ainsi obtenue, soit par exemple pendant une dizaine de minutes, puis à passer ladite pâte à travers une filière pour former des extrudés, par exemple de diamètre compris entre 0,4 et 4 mm. Puis, après séchage pendant quelques minutes à 100 °C en étuve et après calcination, par exemple pendant 2 heures à 400 °C, l'élément éventuel, par exemple le nickel, peut être déposé, par exemple par échange ionique, ledit dépôt étant suivi d'une calcination finale, par exemple pendant 2 heures à 400 °C.

La mise en forme du catalyseur selon l'invention est généralement telle que le catalyseur est de préférence sous forme de pastilles, d'aggrégats, d'extrudés ou de billes, en vue de son utilisation.

La préparation du catalyseur se termine généralement par une calcination, dite calcination finale, habituellement à une température comprise entre 250 et 600 °C, de préférence précédée d'un séchage, par exemple à l'étuve, à une température généralement comprise entre la température ambiante et 250 °C, de préférence entre 40 et 200 °C. Ladite étape de séchage est de préférence menée pendant la montée en température nécessaire pour effectuer ladite calcination.

L'invention concerne l'utilisation dudit catalyseur en dismutation d'hydrocarbures alkylaromatiques, et de préférence la dismutation du toluène pour produire du benzène et des xylènes, et/ou en transalkylation d'hydrocarbures alkylaromatiques, et de préférence la transalkylation d'hydrocarbures alkylaromatiques généralement en C₉⁺ (c'est-à-dire à au moins 9 atomes de carbone par molécule), telle que la transalkylation et/ou la dismutation du toluène et/ou d'alkylaromatiques C₉⁺ pour produire des xylènes. La charge d'un tel procédé peut comprendre de 0 à 100 % d'alkylaromatiques en C₉⁺ et de 0 à 100 % de toluène.

Les conditions opératoires sont généralement les suivantes : une température comprise entre 250 et 600°C et de préférence entre 330 et 500°C ; une pression comprise entre 10 et 60 bar et de préférence entre 20 et 45 bar ; une vitesse spatiale d'alimentation, exprimée en kilogramme de charge introduite par kilogramme de catalyseur et par heure, comprise entre 0,1 et 10 et de préférence entre 0,5 et 4 ; un rapport molaire hydrogène sur hydrocarbures compris entre 2 et 20 et de préférence entre 3 et 12.

Les exemples suivants illustrent l'invention sans toutefois en limiter la portée.

### Exemple 1 : Préparation du catalyseur M1

La matière première utilisée est une zéolithe mordénite, qui possède un rapport atomique Si/AI global égal à 7,6, une teneur pondérale en sodium par rapport au poids en zéolithe mordénite sèche d'environ 3,8 %, un volume de maille élémentaire de 2,759 nm³, et un volume poreux, à l'azote, mesuré à -196°C et à P/Pₒ=0,19 de 0,192 cm³ N2 liquide par gramme.

Cette zéolithe mordénite subit une attaque acide, à l'aide d'une solution d'acide nitrique 5N, à environ 100°C, pendant 4 heures, de manière à extraire partiellement les atomes d'aluminium présents dans la charpente zéolithique de la mordénite. Le volume V de la solution d'acide nitrique engagé (en ml) est égal à 10 fois le poids P de zéolithe mordénite sèche (V/P=10). La zéolithe mordénite ainsi désaluminée est ensuite soumise à un échange ionique dans une solution de NH₄NO₃ 10N à environ 100°C pendant 4 heures de manière à retirer le sodium résiduel.

A l'issue de ces traitements, la zéolithe mordénite sous forme H a un rapport Si/AI atomique global égal à 26,3, une teneur pondérale en sodium par rapport au poids de zéolithe mordénite sèche de 72 ppm, un volume de maille élémentaire de 2,722 nm³, et une capacité d'adsorption en azote mesurée à -196°C et à P/Pₒ=0,19 de 0,204 cm³ N2 liquide/g.

Cette zéolithe mordénite est ensuite mise en forme par extrusion avec un gel d'alumine de manière à obtenir, après séchage et calcination sous air sec, le catalyseur **M1** qui contient en poids 80,2 % de zéolithe mordénite forme hydrogène et 19,8 % d'alumine.

### Exemple 2 : Préparation du catalyseur Ω1

La matière première utilisée est une zéolithe oméga, qui possède un rapport atomique Si/AI global égal à 3,2, une teneur pondérale en sodium par rapport au poids en zéolithe oméga sèche d'environ 5,3 %, un volume de maille élémentaire de 2,196 nm³, et un volume poreux, à l'azote, mesuré à -196°C et à P/Pₒ=0,19 de 0,125 cm³ liquide par gramme.

Cette zéolithe oméga subit tout d'abord une calcination dite sèche à 550°C sous flux d'air et d'azote durant 6 heures. Puis, le solide obtenu est soumis à trois échanges ioniques dans une solution de NH₄NO₃ 10N, à environ 100°C pendant 4 heures pour chaque échange. La zéolithe oméga est alors soumise à un traitement hydrothermique, en présence de 50% de vapeur d'eau à 600°C, durant 4 heures. La zéolithe subit alors une attaque acide, à l'aide d'une solution d'acide nitrique 1N, à environ 100°C, pendant 2 heures, de manière à extraire les espèces aluminiques extra-réseau formées lors du traitement hydrothermique. Le volume V de la solution d'acide nitrique engagé (en ml) est égal à 10 fois le poids P de zéolithe oméga sèche (V/P=10). Puis la zéolithe oméga précédemment traitée est soumise de nouveau à un traitement hydrothermique, en présence de 50% de vapeur d'eau mais cette fois ci à à 700°C, durant 4 heures, puis à une attaque acide, par une solution d'acide nitrique 1,5N à environ 100°C, pendant 4 heures, de manière à extraire les espèces aluminiques extra-réseau formées lors du traitement hydrothermique. Le volume V de la solution d'acide nitrique engagé (en ml) est égal à 10 fois le poids P de zéolithe oméga sèche (V/P=10).

A l'issue de ces traitements, la zéolithe oméga sous forme H a un rapport Si/AI atomique global égal à environ 25, une teneur pondérale en sodium par rapport au poids de zéolithe oméga sèche de 90 ppm, un volume de maille élémentaire de 2,115 nm³, et une capacité d'adsorption en azote mesurée à -196°C et à P/Pₒ=0,19 de 0,206 cm³ N2 liquide/g.

Cette zéolithe oméga est ensuite mise en forme par extrusion avec un gel d'alumine de manière à obtenir, après séchage et calcination sous air sec, le catalyseur **Ω1** qui contient en poids 80,2 % de zéolithe oméga et 19,8 % d'alumine.

### Exemple 3 : Préparation du catalyseur M2

Le catalyseur M1 obtenu à l'exemple 1, est soumis à 3 échanges ioniques avec une solution d'acétate de nickel de manière à introduire 1,0% poids de nickel dans le catalyseur.

Pour cela, le catalyseur M1 est mis en contact avec une solution 0,5 M de Ni(CH₃CO₂)₂ à température ambiante et sous agitation. Entre chaque échange, le solide est séparé de la solution d'imprégnation et lavé abondamment à l'eau permutée. La concentration de la solution d'imprégnation est pour chaque échange ré-ajustée à la concentration de 0,5 mole par litre.

Le solide humide est ensuite séché à 120°C pendant 12 heures, et calciné sous un débit d'air sec à la température de 500°C pendant 1 heure. Le catalyseur **M2** ainsi obtenu contient, en %poids, 79,3 % de mordénite forme hydrogène, 19,6 % d'alumine et 1,1 % de Nickel.

### Exemple 4 : Préparation du catalyseur Ω2

Le catalyseur **Ω1** obtenu à l'exemple 2, est soumis à 3 échanges ioniques avec une solution d'acétate de nickel de manière à introduire 1,0% poids de nickel dans le catalyseur.

Les conditions opératoires des échanges ioniques, du lavage, du séchage et de la calcination sont identiques à celles indiquées dans l'exemple 3.

Le catalyseur **Ω2** ainsi obtenu contient, en % poids, 79,5 % de zéolithe oméga, 19,6 % d'alumine et 0,9 % de Nickel.

Un synopsis des compositions des catalyseurs M1 de l'exemple 1, et **Ω1** de l'exemple 2, M2 de l'exemple 3 et Ω**2** de l'exemple 4 est donné dans le tableau suivant :

| Catalyseurs | Zéolithe **Mordénite** (%poids) | Zéolithe **Oméga** (%poids) | **Nickel** (%poids) | **Alumine** (%poids) |
|---|---|---|---|---|
| **M1** | 80,2 | 0,0 | 0,0 | 19,8 |
| Ω**1** | 0,0 | 80,2 | 0,0 | 19,8 |
| **M2** | 79,3 | 0,0 | 1,1 | 19,6 |
| **Ω2** | 0,0 | 79,5 | 0,9 | 19,6 |

### Exemple 5 : Evaluation des performances des catalyseurs

Les catalyseurs sont mis en oeuvre dans un réacteur à lit fixe, sous pression, dans lequel est introduit la charge qui est constituée de toluène pur.

La comparaison des rendements en (benzène + ethylbenzène + xylènes) obtenus en utilisant deux lits de catalyseur comportant un lit de catalyseur M1 (respectivement M2) et un lit de catalyseur Ω1 (respectivement Ω2), conformément à l'invention, et un lit de catalyseur M1 (respectivement M2) et un lit de catalyseur Ω1 (respectivement Ω2), non conformes à l'invention, est effectuée dans le tableau ci-après :

| Catalyseurs | M1 (non conforme) | Ω1 (non conforme) | M1+Ω1 1^{er} lit 2^{e} lit (conforme) | M2 (non conforme) | Ω2 (non conforme) | M2+Ω2 1^{er} lit 2^{e} lit (conforme) |
|---|---|---|---|---|---|---|
| Température de réaction (°C) | 450 | 450 | 450 | 430 | 430 | 430 |
| Pression totale de réaction (Bar) | 30 | 30 | 30 | 40 | 40 | 40 |
| Rendements % poids (Benzène + Ethylbenzène + xylènes) | 43,4 | 38,9 | 43,6 | 42,2 | 36,9 | 42,5 |

Ainsi, le procédé selon l'invention conduit à des rendements en (Benzène + Ethylbenzène + xylènes) supérieurs.

## Revendications

1. Procédé de dismutation et/ou de transalkylation d'hydrocarbures alkylaromatiques dans une zone réactionnelle comportant au moins deux lits catalytiques comportant chacun un catalyseur différent, l'un des catalyseurs contenant au moins une zéolithe de structure mordénite au moins en partie sous forme acide, et l'autre catalyseur contenant au moins une zéolithe de structure mazzite au moins en partie sous forme acide, chaque catalyseur contenant au moins une matrice et éventuellement au moins un élément choisi dans l'ensemble formé par les groupes lB et VIII de la classification périodique des éléments.

2. Procédé selon la revendication 1, dans une zone réactionnelle comportant au moins deux lits catalytiques comportant chacun un catalyseur différent, l'un des catalyseurs comportant généralement entre 5 et 95% d'une zéolithe de structure mordénite et éventuellement entre 0,01 et 10% d'au moins un élément choisi dans l'ensemble formé par les groupes lB et VIII de la classification périodique des éléments, le complément à 100 % poids consistant en la part de matrice dans ledit catalyseur, et l'autre catalyseur comportant généralement entre 5 et 95% d'une zéolithe de structure mazzite et éventuellement entre 0,01 et 10% d'au moins un élément choisi dans l'ensemble formé par les groupes lB et VIII de la classification périodique des éléments, le complément à 100 % poids consistant en la part de matrice dans ledit catalyseur.

3. Procédé selon l'une des revendications 1 ou 2 tel que, pour chacun des catalyseurs, la matrice est choisie dans le groupe formé par les argiles, la magnésie, les alumines, les silices, l'oxyde de titane, l'oxyde de bore, la zircone, les phosphates d'aluminium, les phosphates de titane, les phosphates de zirconium et les silice-alumines.

4. Procédé selon l'une des revendications 1 à 3 tel que, pour chacun des catalyseurs, ledit élément est choisi dans le groupe formé par Ag, Pt et Ni.

5. Procédé selon l'une des revendications 1 à 4 tel que la zéolithe de type structural mazzite est choisie dans le groupe formé par la zéolithe mazzite gallosilicate, la mazzite, la zéolithe LZ-202, la zéolithe oméga ou la zéolithe ZSM-4.

6. Procédé selon l'une des revendications 1 à 5 telle que la zéolithe de type structural mazzite est la zéolithe oméga.

7. Procédé selon l'une des revendications 1 à 6 tel que la zéolithe de type structural mazzite est pratiquement totalement sous forme acide.

8. Procédé selon l'une des revendications 1 à 7 tel que la zéolithe de type structural mazzite a une teneur en sodium inférieure à 0,6% poids (par rapport à ladite zéolithe), comprend du silicium et de l'aluminium, et a un rapport molaire Si/l qui est compris entre 5 et 100.

9. Procédé selon l'une des revendications 1 à 8 tel que la zéolithe de type structural mordénite est choisie dans le groupe formé par la mordénite et la zéolithe LZ-211.

10. Procédé selon l'une des revendications 1 à 9 telle que ladite zéolithe de type mordénite est la mordénite.

11. Procédé selon l'une des revendications 1 à 10 tel que la zéolithe de type structural mordénite est pratiquement totalement sous forme acide.

12. Procédé selon l'une des revendications 1 à 11 tel que la zéolithe de type structural mordénite a une teneur en sodium inférieure à 1% poids (par rapport à ladite zéolithe), comprend du silicium et de l'aluminium, et a un rapport molaire Si/AI qui est compris entre 5 et 100.

13. Procédé selon l'une des revendications 1 à 12 tel que chacun des catalyseurs est sous forme de pastilles, d'aggrégats, d'extrudés ou de billes.

14. Procédé selon l'une des revendications 1 à 13 tel que les conditions opératoires sont les suivantes : une température comprise entre 250 et 600°C, une pression comprise entre 10 et 60 bar, une vitesse spatiale d'alimentation, exprimée en kilogramme de charge introduite par kilogramme de catalyseur et par heure, comprise entre 0,1 et 10, et un rapport molaire hydrogène sur hydrocarbures compris entre 2 et 20.

15. Procédé selon l'une des revendications 1 à 14 de dismutation et/ou de transalkylation de toluène et/ou d'alkylaromatiques à au moins 9 atomes de carbone par molécule

## Patentansprüche

1. Verfahren zur Dismutation und/oder zur Transalkylierung von alkylaromatischen Kohlenwasserstoffen in einer Reaktionszone, die wenigstens zwei katalytische Betten umfasst, die je einen unterschiedlichen Katalysator aufweisen, und wobei einer der Katalysatoren wenigstens einen Zeolith mit Mordenitstruktur, wenigstens zum Teil in saurer Form, und der andere Katalysator wenigstens einen Zeolith mit Mazzitstruktur, wenigstens zum Teil in saurer Form, enthält, jeder Katalysator wenigstens eine Matrix und gegebenenfalls wenigstens ein Element gewählt aus der Gesamtheit, die durch die Gruppen IB und VIII des Periodensystems der Elemente gebildet ist, enthält.

2. Verfahren nach Anspruch 1, in einer Reaktionszone mit mindestens zwei Katalysatorbetten, die je einen unterschiedlichen Katalysator aufweisen, einer der Katalysatoren generell zwischen 5 und 95 % eines Zeolith mit Mordenitstruktur und gegebenenfalls zwischen 0,01 und 10 % wenigstens eines Elementes gewählt aus der Gesamtheit, die durch die Gruppen IB und VIII des Periodensystems der Elemente gebildet wird, aufweist, und das Komplement auf 100 Gew.-% aus dem Matrizenteil in diesem Katalysator besteht, und der andere Katalysator generell zwischen 5 und 95 % eines Zeolith mit Mazzitstruktur und gegebenenfalls zwischen 0,01 und 10 % wenigstens eines Elementes aufweist, das aus der Gesamtheit gewählt wird, die durch die Gruppen IB und VIII des Periodensystems der Elemente gebildet wird, und das Komplement auf 100 Gew.-% aus dem Matrizenteil dieses Katalysators besteht.

3. Verfahren nach einem der Ansprüche 1 oder 2, derart, dass für jeden der Katalysatoren die Matrix aus der Gruppe gewählt wird, die durch die Tone, Magnesiumoxid, Aluminiumoxide, Siliziumoxide, Titanoxid, Boroxid, Zirkoniumoxid, Aluminiumphosphate, Titanphosphate, Zirkonphosphate und Siliziumaluminiumoxide gebildet wird.

4. Verfahren nach einem der Ansprüche 1 bis 3, derart, dass für jeden der Katalysatoren dieses Element aus der durch Ag, Pt und Ni gebildeten Gruppe gewählt wird.

5. Verfahren nach einem der Ansprüche 1 bis 4, derart, dass der Zeolith mit Mazzitstruktur aus der Gruppe gewählt ist, die durch den Zeolith Mazzitgallosilikat, Mazzit, Zeolith LZ-202, Omegazeolith oder Zeolith ZSM-4 gebildet wird.

6. Verfahren nach einem der Ansprüche 1 bis 5, derart, dass der Zeolith mit Mazzitstruktur Omegazeolith ist.

7. Verfahren nach einem der Ansprüche 1 bis 6, derart, dass der Zeolith mit Mazzitstuktur praktisch vollständig in saurer Form vorliegt.

8. Verfahren nach einem der Ansprüche 1 bis 7, derart, dass der Zeolith mit Mazzitstruktur einen Natriumgehalt von weniger als 0,6 Gew.-% (bezogen auf diesen Zeolith) aufweist, Silizium und Aluminium umfasst und ein Molverhältnis Si/I aufweist, das zwischen 5 und 100 liegt.

9. Verfahren nach einem der Ansprüche 1 bis 8, derart, dass der Zeolith mit Mordenitstruktur aus der durch Mordenit und Zeolith LZ-211 gebildeten Gruppe gewählt ist.

10. Verfahren nach einem der Ansprüche 1 bis 9, derart, dass dieser Zeolith vom Mordenittyp Mordenit ist.

11. Verfahren nach einem der Ansprüche 1 bis 10, derart, dass der Zeolith mit Mordenitstruktur praktisch vollständig in saurer Form vorliegt.

12. Verfahren nach einem der Ansprüche 1 bis 11, derart, dass der Zeolith mit Mordenitstruktur einen Natriumgehalt von weniger als 1 Gew.-% (bezogen auf diesen Zeolith) aufweist, Silizium und Aluminium umfasst und ein Molverhältnis Si/AI zwischen 5 und 100 aufweist.

13. Verfahren nach einem der Ansprüche 1 bis 12, derart, dass jeder der Katalysatoren in Form von Pastillen, Aggregaten, Extrudaten oder als Kugel vorliegt.

14. Verfahren nach einem der Ansprüche 1 bis 13, derart, dass die Betriebsbedingungen die folgenden sind: eine Temperatur zwischen 250 und 600°C, ein Druck zwischen 10 und 60 bar, eine Raumgeschwindigkeit der Einspeisung zwischen 0,1 und 10, ausgedrückt in Kilogramm zugegebene Charge pro Kilogramm Katalysator und Stunde, und ein Molverhältnis Wasserstoff zu Kohlenwasserstoffen zwischen 2 und 20.

15. Verfahren nach einem der Ansprüche 1 bis 14 zur Dismutation und/oder Transalkylierung von Tuol und/oder Alkylaromaten mit wenigstens 9 Atomen Kohlenstoff pro Molekül.

## Claims

1. A process for the dismutation and/or transalkylation of alkylaromatic hydrocarbons in a reaction zone comprising at least two catalytic beds each comprising a different catalyst, one of the catalysts containing at least one zeolite with structure type mordenite, at least partially in its acid form, and the other catalyst containing at least one zeolite with structure type mazzite, at least partially in its acid form, each catalyst containing at least one matrix and, optionally, at least one element selected from the group formed by groups IB and VIII of the periodic classification of the elements.

2. A process according to claim 1, carried out in a reaction zone comprising at least two catalytic beds each containing a different catalyst, one of the catalysts generally comprising between 5% and 95% of a zeolite with structure type mordenite and, optionally between 0.01% and 10% of at least one element selected from the group formed by groups IB and VIII of the periodic classification of the elements, the complement to 100% consisting of the matrix in said catalyst, and the other catalyst generally comprising between 5% and 95% of a zeolite with structure type mazzite and, optionally between 0.01% and 10% of at least one element selected from the group formed by groups IB and VIII of the periodic classification of the elements, the complement to 100% consisting of the matrix in said catalyst.

3. A process according to claim 1 or claim 2 in which, for each catalyst, the matrix is selected from the group formed by clays, magnesia, aluminas, silicas, titanium oxide, boron oxide, zirconia, aluminium phosphates, titanium phosphates, zirconium phosphates and silica-aluminas.

4. A process according to any one of claims 1 to 3 in which, for each catalyst, said element is selected from the group formed by Ag, Pt and Ni.

5. A process according to any one of claims 1 to 4, in which the zeolite with structure type mazzite is selected from the group formed by gallosilicate mazzite zeolite, mazzite, LZ-202 zeolite, omega zeolite, and ZSM-4 zeolite.

6. A process according to any one of claims 1 to 5, in which the zeolite with structure type mazzite is omega zeolite.

7. A process according to any one of claims 1 to 6, in which the zeolite with structure type mazzite is practically completely in its acid form.

8. A process according to any one of claims 1 to 7, in which the zeolite with structure type mazzite has a sodium content of less than 0.6% by weight (with respect to said zeolite), contains silicon and aluminium, and has an Si/AI molar ratio which is in the range 5 to 100.

9. A process according to any one of claims 1 to 8, in which the zeolite with structure type mordenite is selected from the group formed by mordenite, and LZ-211 zeolite.

10. A process according to any one of claims 1 to 9, in which said zeolite with structure type mordenite is mordenite.

11. A process according to any one of claims 1 to 10, in which the zeolite with structure type mordenite is practically completely in its acid form.

12. A process according to any one of claims 1 to 11, in which the zeolite with structure type mordenite has a sodium content of less than 1% by weight (with respect to said zeolite), contains silicon and aluminium, and has an Si/Al molar ratio which is in the range 5 to 100.

13. A process according to any one of claims 1 to 12, in which the catalyst is in the form of pellets, aggregates, extrudates or spherules.

14. A process according to any one of claims 1 to 13, in which the operating conditions are as follows: a temperature which is in the range 250°C to 600°C; a pressure which is in the range 10 to 60 bar; a supply space velocity, expressed in kilograms of feed introduced per kilogram of catalyst per hour, which is in the range 0.1 to 10, and a hydrogen to hydrocarbons molar ratio which is in the range 2 to 20.

15. A process according to any one of claims 1 to 14, for the dismutation and/or transalkylation of toluene and/or alkylaromatics containing at least 9 carbon atoms per molecule.
